# EUROPEAN PATENT APPLICATION

(11) **EP 2 215 975 A1**
(43) Date of publication of application: **11.08.2010**
(21) Application number: 10250217.6
(22) Date of filing: 09.02.2010
(51) Int. Cl.: A61B 17/04

(54) **Fenestrated suture anchor for knotless fixation of tissue**

(30) Priority: 10.02.2009 US 368946; 03.04.2009 US 418391
(71) Applicant: Arthrex, Inc., Naples, FL 34108-1945 (US)
(72) Inventor: Schmieding, Reinhold, Naples, Florida 33963 (US); Dreyfuss, Peter J., Naples, Florida 34112 (US); Sodeika, John A., Naples, Florida 34119 (US)
(74) Representative: Carpmael, Robert Maurice Charles

(57) **Abstract**

A fenestrated suture anchor for fixation of tissue to bone comprises an implant (200), such a swivel implant (200) with a forked tip (250) for capturing a suture. The implant can be secured in a hole in bone by advancing a fixation device (500), such as a cannulated interference screw (500), to engage and fully seat the implant. The cannulated fixation device (500) is provided with a plurality of openings (505) or fenestrations (505) of various dimensions and geometries to provide multiple pathways through the anchor (i.e., though the interior of the body and through the fenestrations) to allow blood to flow to increase the healing zone, for example, for rotator cuff repair, while also promoting bone in-growth.

## Description

### FIELD OF THE INVENTION

The present invention relates to surgical fixation and, more particularly, to methods of conducting anatomical tissue repair, such as ligament repair and reconstruction, using a fenestrated suture anchor.

### BACKGROUND OF THE INVENTION

When soft tissue tears away from bone, reattachment becomes necessary. Various devices, including sutures, screws, staples, wedges, anchors and plugs have been used in the prior art to secure soft tissue to bone. Surgical methods utilizing suture anchors alone are disadvantageous for reattachment of large areas of detached tissue because they often do not allow good tissue to bone contact.

Reattachment of soft tissue to bone typically requires the surgeon to pass suture material through selected tissue, form a plurality of surgical knots extracorporeally and then move the knots into position adjacent the desired tissue to be sutured. In such procedures, the surgeon must manually tie the knots on the suture strands after the suture is threaded through the selected tissues to be sutured. Knot tying during surgery, particularly arthroscopic surgery, is tedious and time-consuming. There is also a tendency for the knots to deform or collapse as the surgeon manually forces the knots down into the proper position. Also, the suture knots often are exposed to abrasion or cutting by sharp or rough areas along the walls of the bone canal into which anchors are typically inserted to provide fixation of tendon to bone.

Accordingly, a need exists for an improved suture anchor for attaching soft tissue to bone which does not require multiple suture knots and which allows the tendon to remain securely in place until the ligaments naturally attach to bone. A need also exists for such a knotless suture anchor for attaching tissue to bone which employs an implant with a fenestrated configuration that promotes healing of tissue.

### SUMMARY OF THE INVENTION

The present invention is a fenestrated swivel suture anchor for knotless tissue fixation for anatomical tissue repair and reconstruction. In one embodiment, the swivel swivel suture anchor is used to capture a suture chain formed of at least two loops formed of high strength suture. A driver is provided direct the tip of the swivel implant to capture loops of the suture chain. The swivel implant is locked and secured in bone by inserting a cannulated fixation device (for example, an interference plug or screw) over the implant. The cannulated fixation device is provided with a plurality of openings or fenestrations of various dimensions and geometries to provide multiple pathways through the device (i.e., though the interior of the body and through the fenestrations) to allow blood to flow to increase the healing zone, for example, for rotator cuff repair, while also promoting bone in-growth.

The present invention overcomes the disadvantages of the prior art, such as those noted above, by providing a swivel implant at the distal end of a driver that securely engages and locks into a cannulated ribbed body of an interference plug or screw. The swivel implant includes a closed aperture for receiving a strand attached to a graft, such that the strand is able to freely slide through the aperture.

In one embodiment of the invention, the strand is passed through the graft at desired points. A cannulated plug or screw is pre-loaded onto a driver provided with a swivel lock twist-in anchor at its distal end. The strand attached to the graft is passed through the aperture of the swivel implant located at the distal end of the driver. The distal end of the driver together with the implant is inserted directly into the bone. The driver may be rotated (in a clockwise direction, for example) to advance a screw over the anchor to complete insertion. The cannulated plug or screw is provided with a plurality of openings or fenestrations of various dimensions and geometries to provide multiple pathways through the device (i.e., though the interior of the body and through the fenestrations) to allow blood to flow to increase the healing zone, for example, for rotator cuff repair, while also promoting bone in-growth

Other features and advantages of the present invention will become apparent from the following description of exemplary embodiments of the invention described with reference to the accompanying drawings.

### BRIEF DESCRIPTION OF THE DRAWINGS

FIGS. 1-8 depict a series of subsequent steps of shoulder repair using a single-row technique with a swivel suture anchor according to the present invention.

FIGS. 9-16 depict a series of subsequent steps of shoulder repair using a double-row technique with a swivel suture anchor according to the present invention.

FIG. 17 illustrates various views of a driver assembly comprising a rod, an inserter member, a threaded gauge and an end piece, according to the present invention.

FIG. 18 illustrates various views of the inserter member of the driver assembly of FIG. 17.

FIG. 19. illustrates various views of the threaded gauge with thumb pad of the driver assembly of FIG. 17.

FIG. 20 illustrates various views of the rod of the driver assembly of FIG. 17.

FIG. 21 illustrates various views of a swivel implant according to the present invention.

FIG. 22 illustrates various views of a fixation device according to a first embodiment of the present invention and used in conjunction with the swivel implant of FIG. 20 and the driver of FIG. 17.

FIGS. 23-28 depict a series of steps of shoulder repair using a plurality of swivel anchor devices according to the present invention.

FIG. 29 illustrates various views of the driver assembly of the present invention.

FIGS. 30 and 31 illustrate the swivel implant and traction suture.

FIG. 32 is an enlarged view of the fixation device (cannulated screw) used in the present invention.

FIG. 33 illustrates various views of a swivel anchor with a metal tip which avoids the need to pre-drill a hole in bone.

FIGS. 34 and 35 provide additional illustrations of the swivel anchor assembly of the present invention, and the swivel anchor inserted in a bone socket, respectively.

FIGS. 36-39 illustrate various views of a fixation device with fenestrations and used in conjunction with the swivel anchor assembly.

### DETAILED DESCRIPTION OF THE INVENTION

The present invention provides a fenestrated swivel suture anchor for knotless fixation of tissue. In one embodiment, the swivel suture anchor is used with a suture chain formed of multiple loops of suture, preferably high strength suture. The loops of the chain are captured by mounting the swivel implant on a driver. The driver is also used to engage and lock the swivel implant in a hole in bone by inserting a cannulated fixation device (for example, an interference plug or screw) over the anchor.

The present invention is used in a method for knotless fixation of anatomical tissue during surgical applications by employing a swivel suture anchor. The term "chain" is used in the specification and claims to refer to exemplary embodiments of the invention. A "chain" in this context refers broadly to a suture construct formed of a series of suture loops. The loops can be, but need not be, interlinked. In this manner, the term "chain" as used in this application includes, but need not be limited to, the commonly understood definition in which links or rings are fitted into one another. Rather, the chains of the present invention can include two or more loops that are connected together. Each loop preferably has a fixed perimeter. The suture can be interlaced, rather than knotted, as described further below, to establish and maintain loop geometry. Preferably, all loops are similar in size.

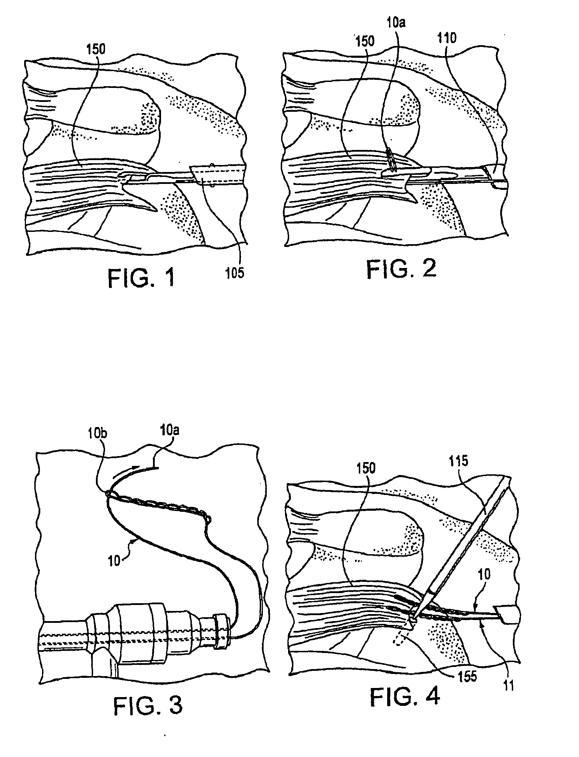

Referring now to the drawings, where like elements are designated by like reference numerals, FIGS. 1-16 illustrate two surgical procedures of approximating tissue (for example, a torn tendon) to bone according to exemplary embodiments of the present invention. For simplicity, the invention will be described below with reference to fixation of the rotator cuff; however, the invention is not limited to this exemplary embodiment and has applicability to fixation of tissue in general, such as, for example, fixation of torn tendons in ACL reconstructions of the knee or in fixation of any tendon to bone.

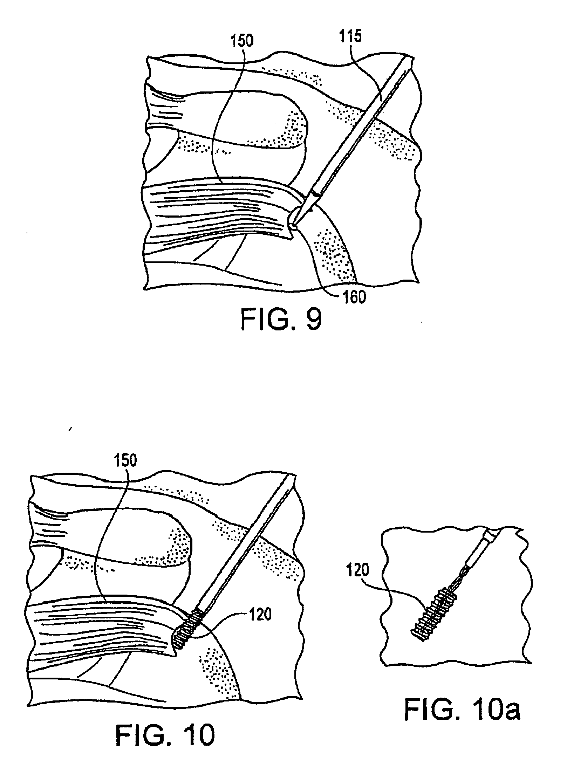

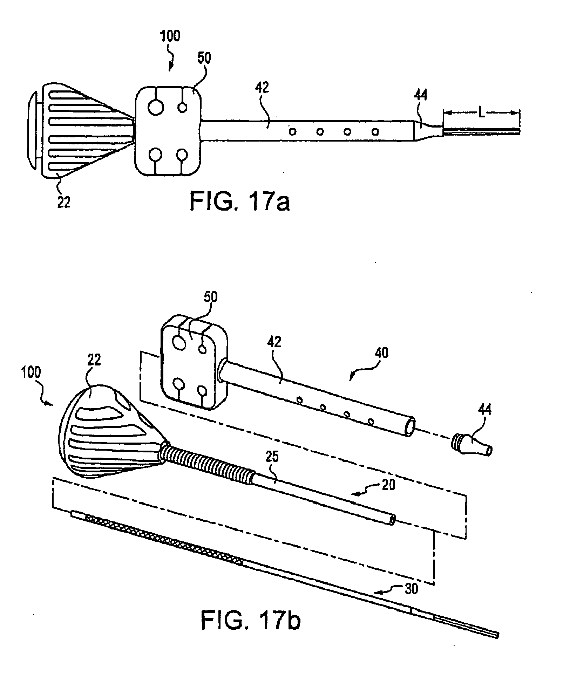

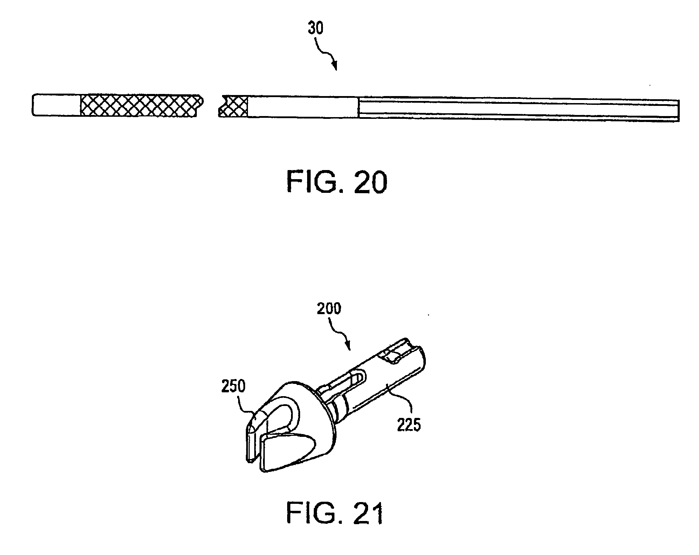

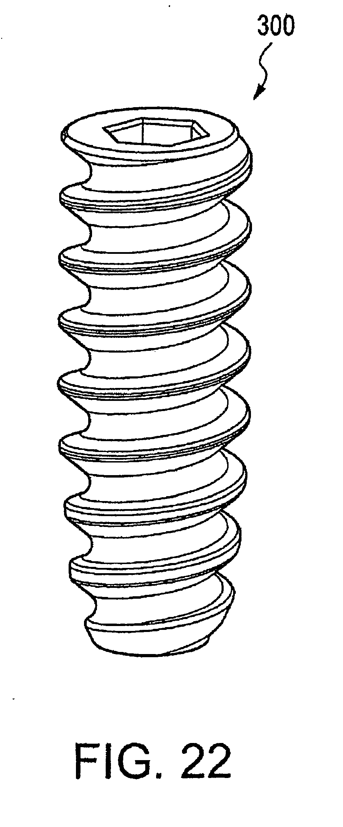

Method steps of tissue fixation using the fenestrated suture anchor of the present invention are depicted schematically in FIGS. 1-16. As described in more detail below, FIGS. 1-8 illustrate sequential steps during a rotator cuff repair using a knotless single row technique and FIGS. 9-16 illustrate sequential steps during a rotator cuff repair using a knotless double row technique. Both techniques will be described with reference to driver assembly 100 (FIGS. 17-20), swivel lock anchor 200 (FIG. 21) and fixation device 300 (FIG. 22).

Although the single and double row techniques are described below with reference to specific instruments sold by Arthrex, Inc., the assignee of the present application, and with reference to a series of specific steps and devices used during the surgical tissue fixation, it should be understood that the invention is not limited to the specific disclosure of these embodiments and contemplates modifications and variations that are apparent to those skilled in the art. Accordingly, the invention must not be limited to the specific embodiments detailed below, as they are only exemplary in nature.

### KNOTLESS SINGLE ROW REPAIR TECHNIQUE - FIGS. 1-8

FIGS. 1-8 illustrate a series of subsequent steps in shoulder repair for a single-row repair technique using a swivel suture anchor according to the present invention.

The mobility of the tear is assessed using a suture retriever/tissue grasper 105, such as the Arthrex KingFisher^{™} Suture Retriever/Tissue Grasper, as shown in FIG. 1. In the case of a large U-shaped tear, margin convergence suturing may be required before approximation. Then, a shaver, high-speed burr, or chondro pick is used to prepare a bleeding bone bed.

As shown in FIG. 2, the non-linked, free end 10a of a suture chain 10, such as the Arthrex FiberChain, is passed through the rotator cuff 150 using a suture passer 110, such as the Arthrex Scorpion^{™} Suture Passer, through a cannula, such as an Arthrex 5.75 mm Crystal Cannula. Next, the suture is retrieved through the same cannula.

Then the free end 10a of suture chain 10 is passed through the terminal link 10b at its opposite end, as shown in FIG. 3. The loop 10b is cinched down by pulling on the free end 10a of the suture chain 10. The tip of the cannula may be used to help seat the suture chain loop securely against the rotator cuff 150. A suture retriever/tissue grasper 105 may be useful to ensure that the loop has been fully tightened. The second suture chain 11 is passed through the rotator cuff 150, the free end passed through the terminal loop, and the loop cinched in the same manner as for the first suture chain 10.

As shown in FIG. 4, the suture chain 10, 11 is used as a traction suture to determine the desired anchor location adjacent to the rotator cuff margin and a bone socket 155 is punched through a superolateral percutaneous portal with a punch 115, such as the Arthrex 5.5 mm Bio-Corkscrew FT Punch. A second bone socket 155 is also formed in the same manner for the second fiber chain 11.

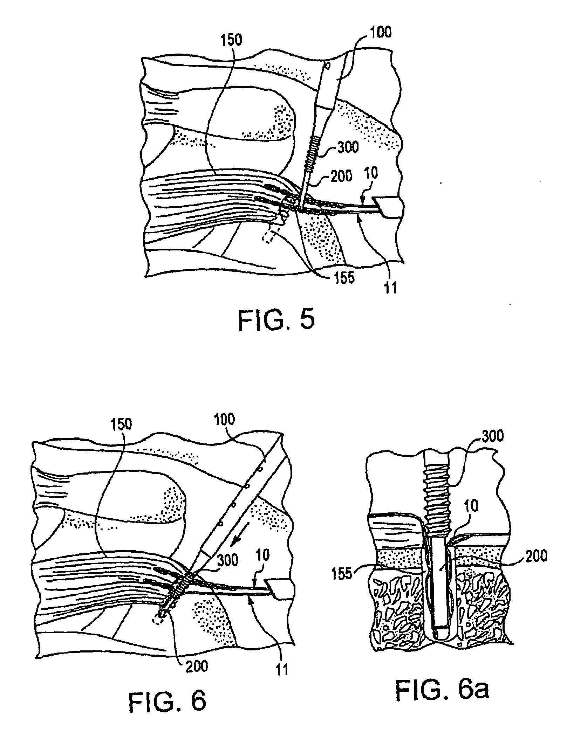

As shown in FIG. 5, both suture chain ends 10a, 11a are retrieved through the lateral portal. The swivel implant 200 of the present invention is introduced through the percutaneous superolateral portal and captures the third link 10c from the free margin of the rotator cuff 150. The link 10c is captured by the forked anchor tip 250 of the anchor 200 (see FIG. 21). In a preferred embodiment, each link in the suture chain 10 is approximately 6 mm in length; therefore, since the total length of the swivel implant 200 is 18 mm, capturing the third link 10c from the cuff edge will usually position the cuff directly at the edge of the bone socket 155 and perfectly tension the suture chain 10 and the rotator cuff 150 segment that it spans, when the anchor tip 250 pushes the suture chain 10 to the bottom of the bone socket 155.

The tissue tension is then evaluated, as shown in FIG. 6. The driver assembly 100 is advanced into the bone socket 155 and the suture chain 10 is pushed toward the bottom of the socket 155 until the anchor body 225 contacts the bone. If the tension is not adequate, the driver 100 is removed from the bone socket 155 by pulling on the free end of the suture chain 10 (to release any wedging of the swivel-tip 250) at the same time that the driver assembly 100 is withdrawn. Then the adjacent, more proximal link is instead captured. If the tension is too great to fully insert the driver 100 to the bottom of the bone socket 155, the driver 100 is removed and the adjacent, more distal link is captured. Then the driver 100 is reinserted to the base of the bone socket. The forked tip 250 of the swivel implant 200 is held to the driver with a zero retention suture cleated at the driver's proximal end.

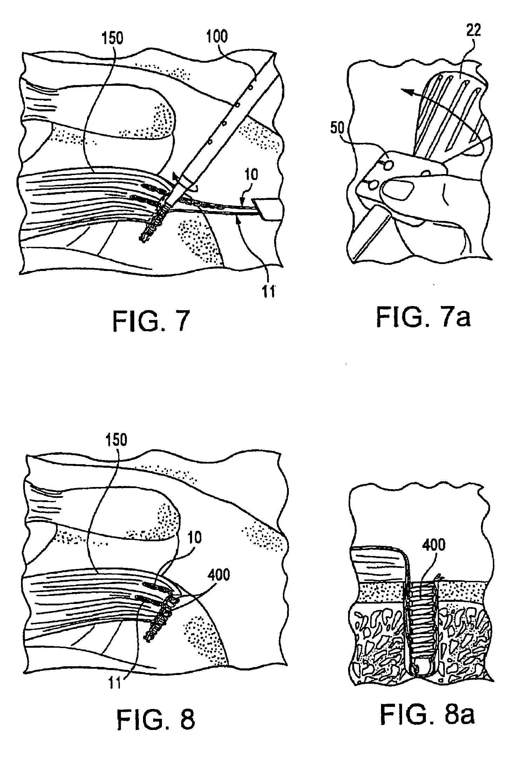

Fixation device 300, such as a screw, is advanced by driver assembly 100 by holding the thumb pad 50 as the inserter handle 22 is turned clockwise, as shown in FIG. 7. When the swivel anchor construct 400 is fully seated, the shaft 225 of the swivel implant 200 is fully engaged by the fixation device 300 to optimize the stability of the swivel implant construct 400. The swivel anchor construct 400 comprises swivel implant 200 and fixation device 300. The tip retention suture is unwound from the cleat at the back of the driver handle 22 and the driver assembly 100 is removed. Then, one limb of the retention suture is pulled to fully remove it from the implant.

The insertion steps are repeated for the second swivel anchor construct 400 to obtain the final suture/anchor construct, shown in FIG. 8. The free suture ends 10a, 11a are cut with a suture cutter, such as the Arthrex open ended FiberWire Suture Cutter, so that they are flush with the edge of the bone socket 155.

### KNOTLESS DOUBLE ROW REPAIR TECHNIQUE - FIGS. 9-16

FIGS. 9-16 illustrate a series of subsequent steps in shoulder repair for a double-row repair technique using a swivel suture anchor according to the present invention.

The mobility of the tear is assessed using suture retriever/tissue grasper. In the case of a large U-shaped tear, margin convergence suturing may be required before approximation of the tendon. A shaver, high-speed burr, or chondro pick is used to prepare a bleeding bone bed. Then, as shown in FIG. 9, pilot holes 160 are prepared for two suture anchors 120, such as Arthrex Bio-Corkscrew FT suture anchors, that will comprise the medial row through a percutaneous superolateral portal. A punch 115, such as the Arthrex 5.5 mm Bio-Corkscrew FT Punch, is advanced to the laser line at a 45° "deadman" angle, adjacent to the articular margin of the humerus to form the pilot holes 160. Tapping is seldom, but may be, necessary.

As shown in FIG. 10, both suture anchors 120 are placed. In a preferred embodiment, the suture anchors 120 come preloaded with suture chain 10.

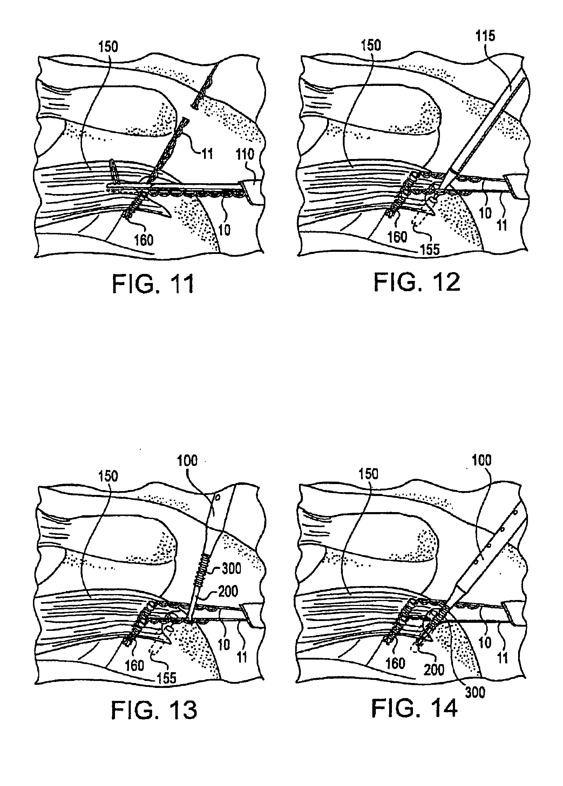

Then, as shown in FIG. 11, the suture leader is retrieved from one of the suture chain 10 strands through the lateral portal and is loaded onto a suture passer 110, such as the Arthrex Scorpion Suture Passer. The suture chain 10 is passed through the rotator cuff 150 approximately 15 mm from the free margin of the rotator cuff 150. This step is then repeated for the second suture chain 11.

As shown in FIG. 12, both suture chain suture ends 10a, 11a are retrieved through the lateral portal and are tensioned to bring the cuff into contact with the medial portion of the footprint. The tip of the cannula may be used to push the tendon against the footprint. Then two bone sockets 155 are formed for the lateral row swivel implants 200 using a punch 115. These two bone sockets 155 should be adjacent to the lateral margin of the cuff 150 when the cuff 150 is appropriately tensioned by the two previously placed suture chains 10, 11.

As shown in FIG. 13, the swivel implant 200 is introduced through the percutaneous superolateral portal, capturing the third link 10c from the free margin of the rotator cuff 150. The link 10c is captured by the forked anchor tip 250 of the swivel implant 200 (see FIG. 21). In a preferred embodiment, each link in the suture chain 10, 11 is approximately 6 mm in length; therefore, since the total length of the swivel implant 200 is 18 mm, capturing the third link from the cuff edge will usually position the cuff directly at the edge of the bone socket 155 and perfectly tension the suture chain 10 and the rotator cuff segment that it spans when the anchor tip 250 pushes the suture chain 10 to the bottom of the bone socket 155.

The tissue tension is then evaluated, as shown in FIG. 14. The driver assembly 100 is advanced into the bone socket 155 and the suture chain 10 is pushed toward the bottom of the socket 155 until the anchor body 225 contacts the bone. If the tension is not adequate, the driver is removed from the bone socket by pulling on the free end of the suture chain 10 (to release any wedging of the swivel-tip 250) at the same time that the driver assembly 100 is withdrawn and the adjacent, more proximal link is instead captured. If the tension is too great to fully insert the driver to the bottom of the bone socket 155, the driver assembly 100 is removed and the adjacent, more distal link is instead captured. Then the driver 100 is reinserted to the base of the bone socket 155.

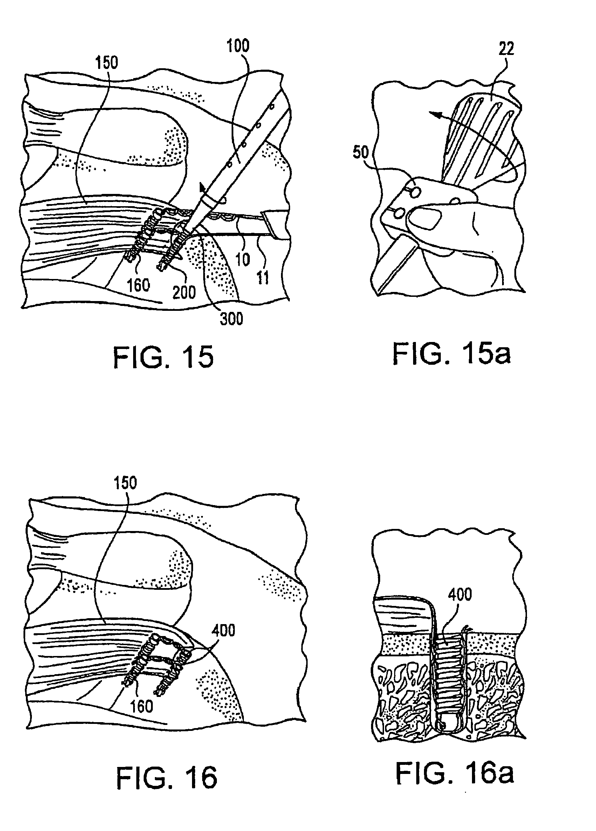

Fixation device 300, e.g., a screw, is advanced by holding the thumb pad 50 as the inserter handle 22 is turned clockwise, as shown in FIG. 15. When the swivel anchor 400 is fully seated, the shaft 225 of the swivel implant 200 is fully engaged by the body of the fixation device 300 to optimize the stability of the swivel implant construct 400. As previously stated, the swivel anchor construct 400 comprises anchor 200 and fixation device 300. The tip retention suture is unwound from the cleat at the back of the driver handle 200 and the driver 100 is removed. One limb of the retention suture is pulled to fully remove it from the implant.

The steps of FIGS. 13-15 are then repeated for the second swivel implant construct 400 to obtain the final construct, shown in FIG. 16. The free suture ends are cut with a suture cutter so that they are flush with the edge of the bone socket 155.

In addition to the above exemplary embodiments, the invention may also be used for a side-to-side closure of soft tissue using a suture chain 10. This method includes using the suture chain 10 to perform the side-to-side closure of the soft tissue with a final step of anchoring a chain link at the lateral aspect of the repair to bone using an anchor, such as anchor 200. The anchoring step is the same as that illustrated in the first two embodiments. Furthermore, if the soft tissue split to be repaired overlies bone for its entire length, a suture anchor, similar to suture anchor 120, may be inserted into bone at the medial aspect of the repair, a side-by-side margin convergence is implemented using the suture chain in a "shoelace-type" stitch, and the suture chain is anchored at the lateral aspect of the repair to bone using an anchor, such as anchor 200.

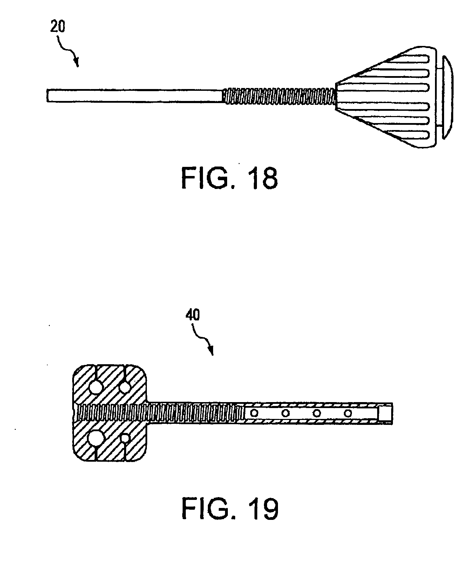

Reference is now made to FIGS. 17-22, which illustrate details of the driver assembly 100 (FIGS. 17-20), the swivel implant 200 (FIG. 21) and the fixation device 300 (FIG. 22). As illustrated in FIGS. 17-20, driver assembly 100 comprises a cannulated driver 20 including shaft 25 and inserter handle 22; tube or rod 30 that passes slidably and rotatably through the cannulated driver 20; threaded gauge 40 including a cannulated body 42 and thumb pad 50; and tip 44. FIG. 17 illustrates the driver assembly 100 both in assembled (A) and unassembled (B) form. FIG. 18 provides a more detailed view of the cannulated driver 20 of the driver assembly 100. FIG. 19 provides a more detailed view of the threaded gauge 40 of the driver assembly 100. FIG. 20 provides a more detailed view of the rod 30 of the driver assembly 100.

FIG. 21 illustrates various views of swivel implant 200. During installation of swivel implant 200, the implant body 225 is assembled onto the operational end of the driver 100. The implant tip 250 is threaded or otherwise attached onto the tip of thin rod or tube 30. As detailed above, forked tip 250 is used to capture a suture chain 10, 11 (that has been laced through a shoulder ligament, as shown in the two exemplary embodiments above) for installation into a pre-drilled hole 155 in bone. The implant 200 has a threaded body 225 and a detachable forked tip 250. The forked tip 250 may be rotatably attached to the implant body 225. This enables rotational insertion of the implant 200 without causing excessive twisting and knotting of the suture chain 10, 11 by the forked tip 250.

FIG. 22 illustrates various views of a fixation device 300 (for example, a cannulated interference screw) that is employed in conjunction with the driver assembly 100 and the swivel implant 200. Preferably, the fixation device 300 is preloaded on the driver assembly 100. As described above with reference to the two exemplary embodiments, the fixation device 300 is advanced into the bone socket 155 by holding the thumb pad 50 as the inserter handle 22 is turned clockwise. When the fixation device 300 is fully seated, the shaft 225 of the forked swivel implant 200 is fully engaged by the fixation device 300 to optimize the stability of the swivel implant construct 400. As previously stated, the swivel implant construct 400 is composed of anchor 200 and fixation device 300.

In the two exemplary embodiments detailed above, suture anchor 400 is includes a threaded fixation device. However, suture anchor 400 may also include a ribbed (push-in) fixation member such as an Arthrex Push-Lock™- anchor, but including a forked tip in order to capture a given link of the suture chain. Alternatively, the suture chain 10, 11 passed through the tissue can be secured either using a single anchor or a plurality of anchors. In addition, various anchors, such as those noted above and others, may be used interchangeably with only slight variations in the above procedure. For example, the suture chain 10, 11 can be secured by capturing two of the chain loops in forked tines prior to insertion of the anchor or anchors.

Further, regular suture may be used in addition to the suture chains of the present invention. In this case, the first suture anchor 120 of the double row embodiment will be pre-loaded with regular suture (for example, the Arthrex BioCorkscrew™ or Arthrex BioCorkscrew-FT™, disclosed in U.S. Application Publication No. 2007/0060922). In an exemplary embodiment using regular suture, the technique is similar to the one described above, except that the lateral fixation is accomplished by capturing the suture limbs (rather than chain-links) in the fork of the implant 200 and tensioning the suture as the implant 200 is placed. This relies on interference fixation of the suture between the implant 200 and the bone.

As described above, the swivel anchor and suture chain assembly of the present invention has application in surgical tissue repair, for example, in conjunction with one or more bone anchors. Tension on repair constructs is adjustable through selection of the chain link or links to be snagged by a bone anchor.

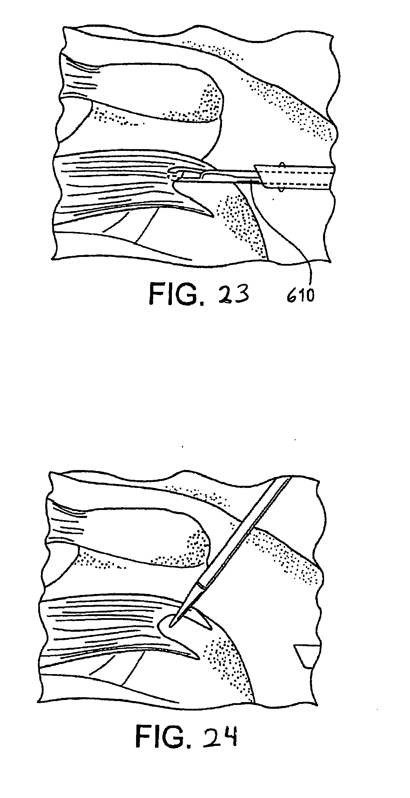

FIGS. 23-35 describe another embodiment.

FIG. 23 illustrates a side view of a human shoulder of a patient undergoing a rotator cuff repair in accordance with an exemplary embodiment of the present invention. The patient may be positioned in the beach chair position using the Arthrex Beach Chair Lateral Traction Device or in a lateral decubitus position using the Arthrex 3-Point Shoulder Distraction System. Access to the subacromial space is facilitated with a variety of cannulas.

First, and as illustrated in FIG. 23, the mobility of the tear is assessed using, for example, a tissue grasper 610 such as the Arthrex KingFisher™ Suture Retriever/Tissue Grasper, to determine whether a U or L-shaped component exists. Where large tears extend to the superior aspect of the glenoid, margin convergence suturing is performed to reduce volume and strain on the repair. Subsequently, the length and width of the rotator cuff footprint is assessed and a bleeding bed for enhanced tendon to bone healing may be formed. This may be accomplished with a burr to perform a light dusting of the greater tuberosity, or by using a chondro pick to microfracture the footprint and maximize vascular channels.

FIG. 24 illustrates the preparation of two pilot holes for two swivel anchors that will be inserted in the medial row. A punch may be employed adjacent to the articular margin of the humerus and at about 45° angle to form the two pilot holes.

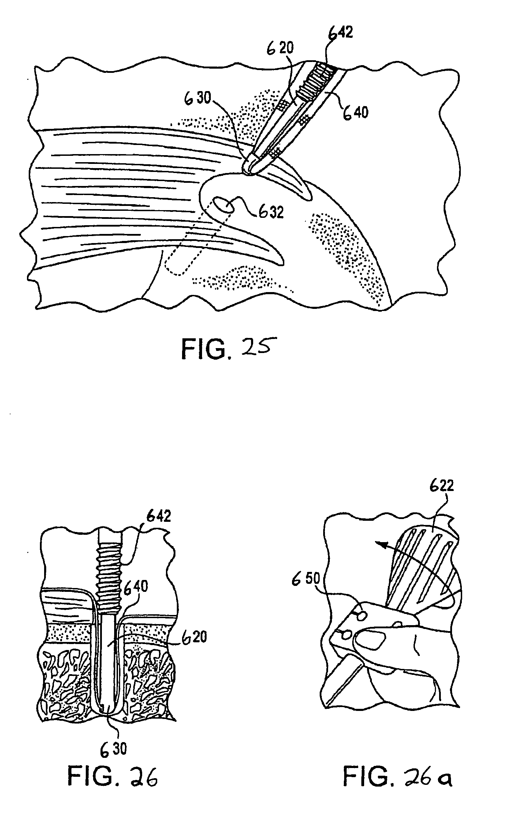

Subsequent to the formation of the pilot holes, and as shown in FIGS. 25 and 26, a swivel implant 630, loaded with a strand of suture tape 640, preferably Arthrex FiberTape, is placed in the medial pre-formed hole 632. However, the anchor of the present invention can be used with any type of flexible material or suture. The driver is then rotated to advance screw 642 down shaft 620 to secure the implant and suture in the bone hole. More specifically, as shown in FIG. 26a, the screw 642 is advanced by holding thumb pad 650 as the driver handle 622 is turned clockwise. An Arthrex FiberLink and an Arthrex Scorpion suture passer 644, are used to shuttle both tails of the suture tape through the rotator cuff 634 simultaneously. This procedure is followed for both medial swivel anchors.

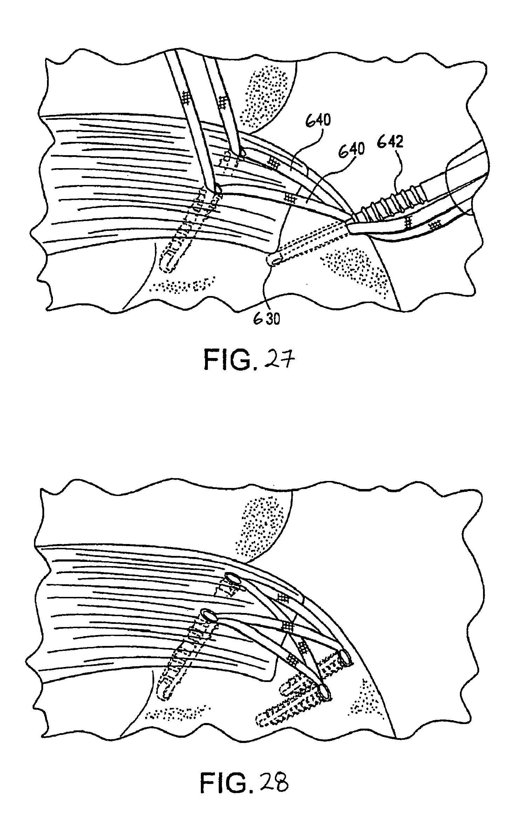

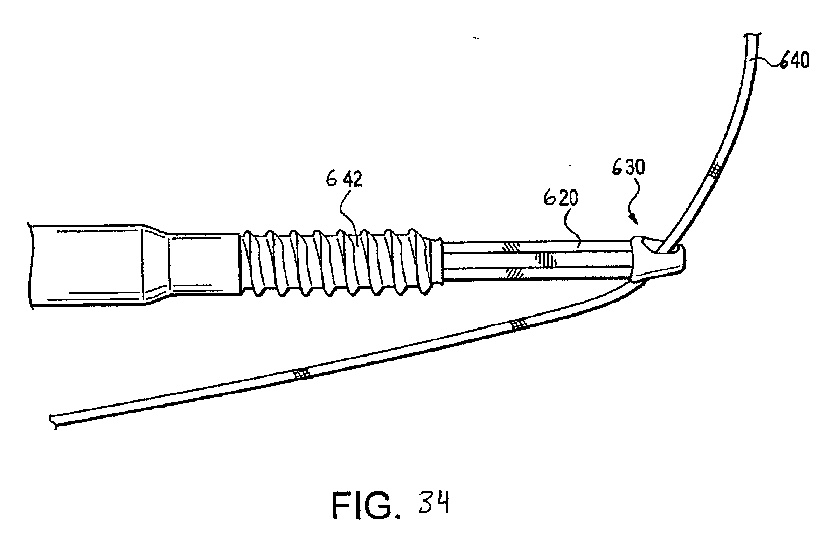

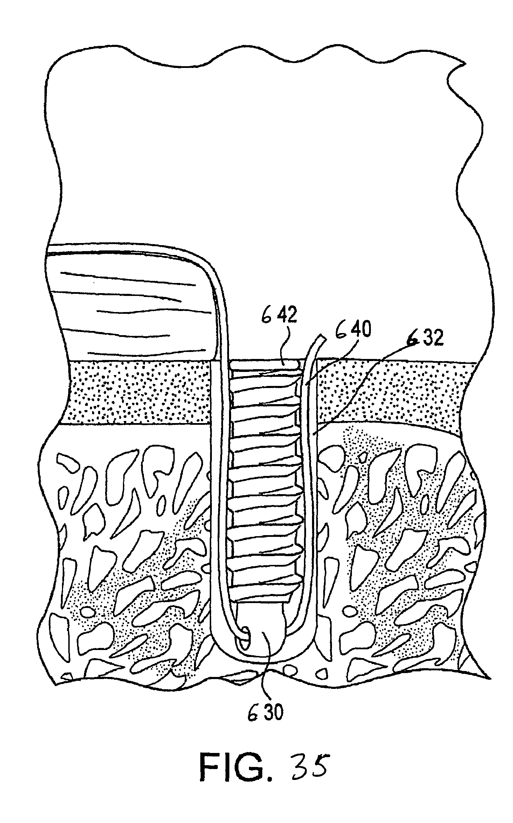

Referring to FIG. 27, one tail of suture tape 640 from each medial swivel anchor is retrieved and loaded through the eyelet of another swivel implant 630, and that implant is installed in then inserted into a preformed lateral bone socket. The tension of the suture tape 640 is adjusted if necessary. The swivel anchor driver is then rotated in clockwise direction as before to advance the screw 642 over the implant to complete insertion. This step is repeated in another lateral bone socket with the other tails of suture tape from each medial anchor. The tails of the suture tape 640 are then cut, one at a time, to complete the construct as shown in FIG. 28. FIGS. 34 and 35 provide additional illustrations of the swivel anchor assembly and the anchor inserted in a bone socket, respectively.

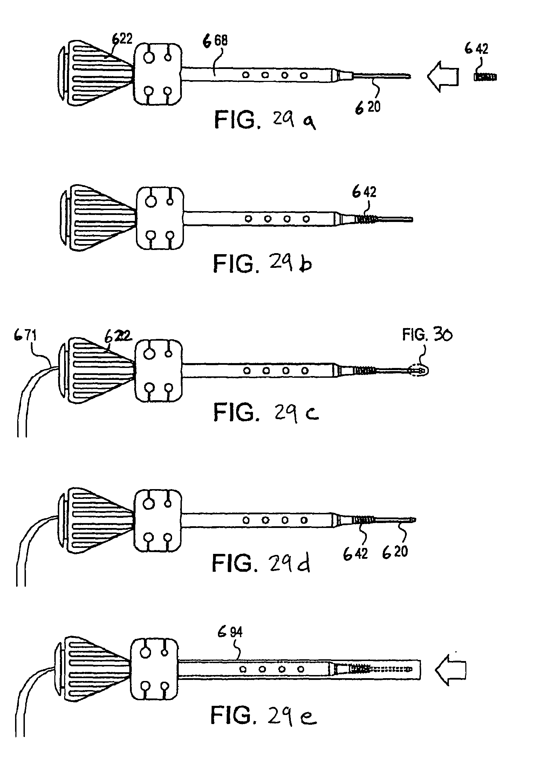

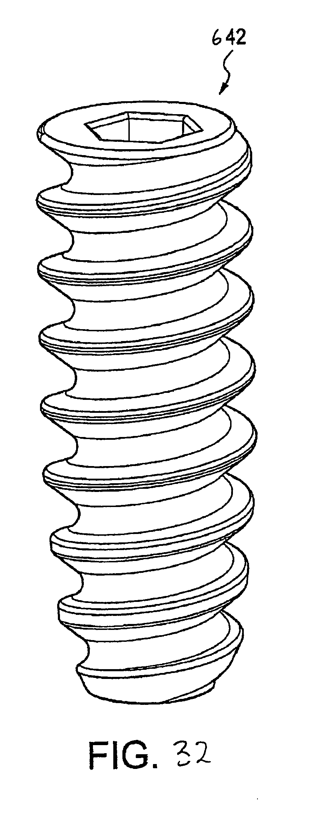

The swivel anchor and instruments of the present invention are now described in greater detail. As shown in FIGS. 29(a)-(f), a driver 668 is used to install the knotless fixation devices with a swiveling implant. Driver 668 features a thin cannulated rod 620 passing slidably and rotatably through a cannulated driver assembly. The tip of thin cannulated rod 620 is adapted to accept swivel anchor implant 642 within the cannulation at its tip, preferably via a snap fit. Cannulated rod 620 has a hexagonal outer surface for receiving anchor body (preferably a screw) 642 having a corresponding cannulation. FIG. 32 illustrates a detailed view of the cannulated screw 642.

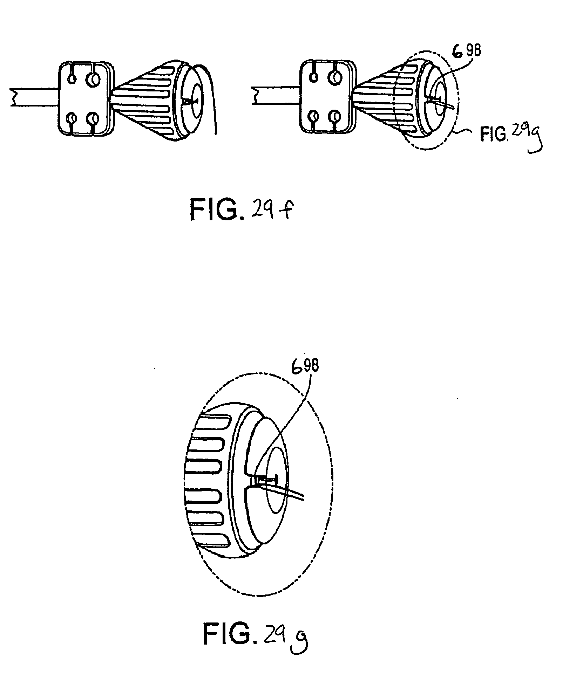

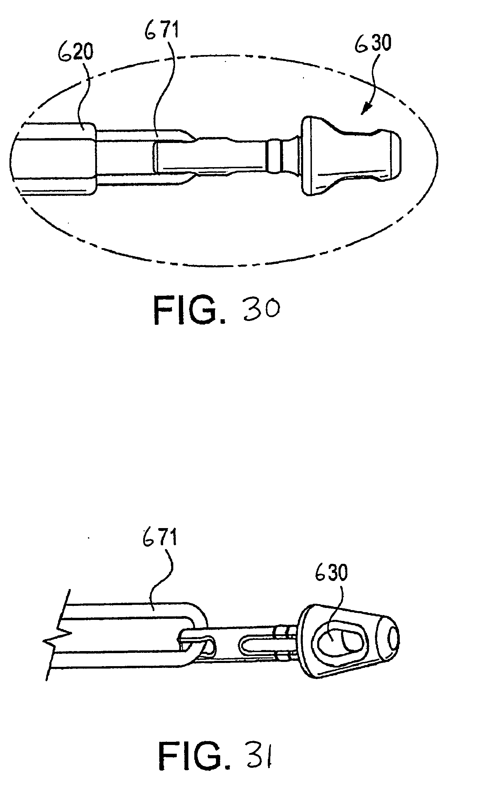

During installation of the knotless anchor having a swiveling implant 630, the screw 642 is first inserted onto cannulated rod 620 of the driver 668. As shown in FIGS. 29(a) and (b), screw 642 is loaded onto rod 620 and then fully seated on the shaft end of the driver. FIG. 29(c) illustrates the swivel anchor implant 630. As shown in FIGS. 30-31, traction sutures 671 extending from the proximal end of the swivel anchor implant 630 are threaded through the cannulation of the driver 668 (see also FIG. 29(c)). These traction sutures 671 prevent inadvertent separation of the implant 630 from the driver during insertion, but they can be used subsequently for additional tie-down of the tendon after the driver is removed. Subsequently, the swivel anchor implant 630 is seated on the driver tip and until advanced until it snaps onto place (FIG. 29(d)). A protective tube 694 (FIG. 29(e)) may be placed over the tip of the assembly for shipping purposes. The traction sutures 671 may be looped around the driver handle, as shown in FIGS. 29(f) and (g), and secured in a cleat 698 to prevent the implant 630 from becoming prematurely detached from the driver.

The knotless fixation device of the present invention advantageously minimizes or eliminates the need to tie knots. The use of such a swivel anchor also provides secure fixation of the suture construct - the secure suture construct results from the suture being pushed into a hole and held tightly by an anchors.

In the preferred embodiment of the present invention, as mentioned above, suture tape is used with the swivel anchor to fix tissue to bone. However, the swivel anchor of the present invention can be used with any type of flexible material or suture. In another preferred embodiment, an allograft or biological component may be used instead of suture or tape. The allograft or biological component may be comprised of tendon or pericardium, for example, which provides improved tissue repair. In yet additional embodiments, any combination of suture, suture tape, and allograft or biological component may be employed, depending on the characteristics of the specific surgical repair and/or as desired.

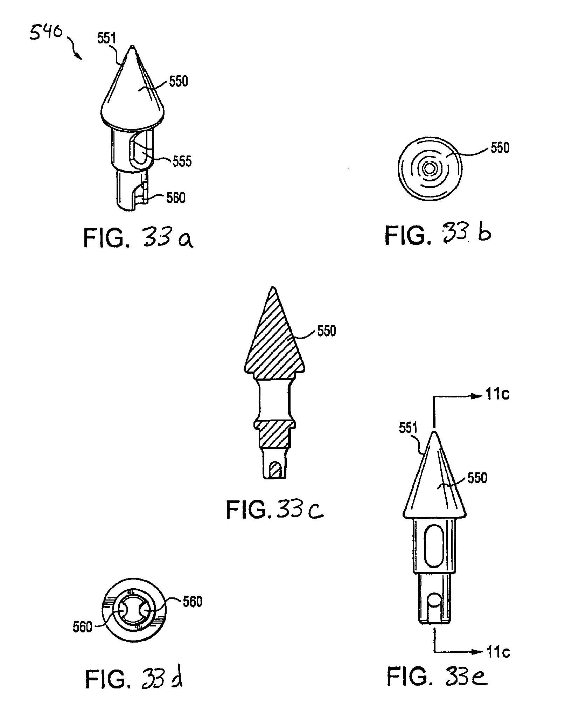

FIG. 33 illustrate a swivel implant 540 which is provided with a pointed metal tip to facilitate insertion of the implant without the need to pre-drill or pre-form a hole in the bone. The conical configuration of the most distal end pointed tip 550 allows the implant to undergo a self-punching operation, eliminating any need to pre-drill a hole in the bone. The conical configuration of the most distal end of the pointed tip implant 550 also provides suture fixation strength, as well as accelerated graft/tendon healing to bone. The pointed tip implant 550 may be detachable from the driver.

As illustrated in FIGS. 33(a)-(e), pointed tip implant 540 is provided with a metal tip 550 and an eyelet or aperture 555 for receiving suture or suture tape. Pointed tip implant 550 is also provided, at its most distal end, with a conical portion 551 which allows direct advancement of the implant (by simply tapping the device with a mallet, for example) without the formation of a pilot hole in bone. Preferably, the conical portion 551 of the implant is formed of titanium or titanium alloy. In a preferred embodiment, eyelet or aperture 555 is also formed of titanium or similar material, to withstand impaction forces during the graft fixation procedure.

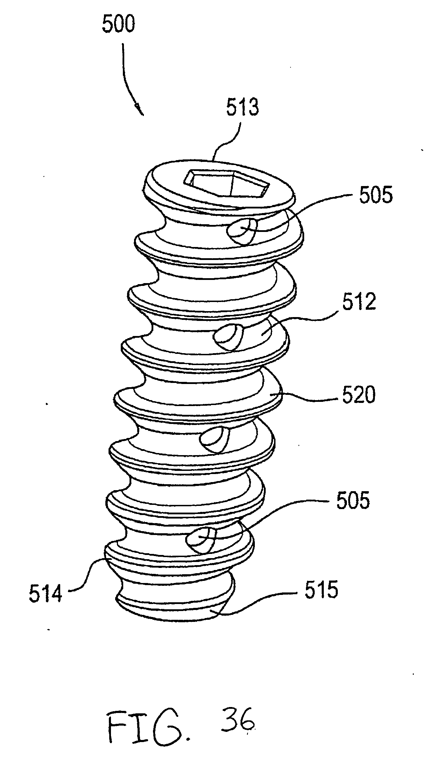

FIGS. 36-39 illustrate various views of another exemplary fixation device 500 of the present invention that is employed in conjunction with the driver assembly 100 and the swivel implant 200 and 540. The fixation device 500 is similar to the fixation device 300 of FIG. 22, but differs from it in that the fixation device 500 is provided with a plurality of openings or fenestrations 505 provided on the outer surface of the body of the device. The openings or fenestrations 505 may have various dimensions and geometries provide multiple pathways for w blood to pass through the device (i.e, through the fenestrations and up through the cannulation) and, therefore, to the repair site to promote healing. The fenestrations also promote in-growth of bone. The decreased mass of the device 500 (resulting from the fenestrations) further promotes healing and in-growth.

Preferably, the fixation device 500 is preloaded on the driver assembly 100. As described above with reference to the three exemplary embodiments, the fixation device 500 is advanced into the bone socket 155 by holding the thumb pad 50 as the inserter handle 22 is turned clockwise. When the fixation device 500 is fully seated, the shaft 225 of the forked swivel implant 200 is fully engaged by the fixation device 500 to optimize the stability of the swivel anchor construct (composed of implant 200 and fixation device 500).

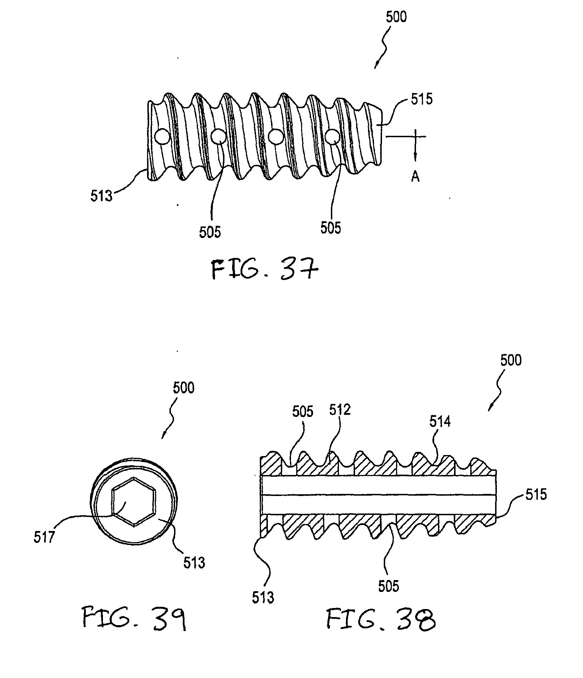

As illustrated in FIGS. 36-39, the fixation device 500 includes a cannulated body 512 in the form of a tapered cylinder having a proximal end 513 and a distal end 515. A continuous thread 520 wraps around cannulated body 512 in a clockwise direction, as shown. As shown in FIG. 25, the distal end 515 of the interference screw 500 terminates in an exposed, flat surface provided with an opening 516. The proximal end 513 of the interference screw 500 terminates in a drive socket 517 that allows a driver to seat snuggly in the drive socket to allow manipulation and installation of the interference screw into the bone socket, while fully engaging the shaft of the swivel implant 200 (as detailed above with reference to interference crew 300). In another embodiment, the proximal end 513 of the fixation device 500 terminates in a drive socket 517 that allows a driver to seat snuggly in the drive socket to allow manipulation and installation of the interference screw into the bone socket, while fully engaging the shaft of the swivel anchor 630 or swivel implant 540 (as detailed above with reference to interference screw 642). As shown in FIG. 39, drive socket 517 may be configured to be used with a traditional hex drive screwdriver. Although the drive socket 517 has been described as having hexagonal shape, the drive socket may also have a Delta drive configuration or a cruciform shape, among others, that allows the driver to rotationally engage the interference screw, to turn simultaneously with the driver.

The fixation device 500 of the present invention may be formed of a biocompatible and/or biosorbable material. Preferably, screw 500 is formed of a bioabsorbable material, such as poly-(L-lactic acid) (PLLA), poly-(D,L-lactide), and poly glycolic acid (PGA), for example, or other bioabsorbable, non-metallic materials, which may be especially tailored for hardness, tensile strength and compressive strength. Alternatively, fixation device 500 may be formed of titanium, titanium alloy, stainless steel or stainless steel alloy. Other biocompatible materials which could be used include plastics, allograft bone and inert bone substitute materials.

A growth material may be advanced through the cannulated driver and into the screw 500 by employing a plunger, for example. As the driver is pulled out, the plunger pushes the flow material through the cannulation of the driver and into the body of the screw 500. The growth material will subsequently harden to allow better fixation of the interference screw 500 against the bone and the shaft of the swivel implant 200 and 540.

The growth material may be any solid, semi-solid, viscous, flowable, gel or elastic composition or mixture that allows its easy manipulation and insertion into the body 512 of the interference screw 500. The growth material may contain growth factors such as autogenous growth factors, for example platelet-rich plasma (PRP), optionally in combination with hyaluronic acid (HY acid) and/or with a coagulant such as thrombin.

The term "growth factor" as used in the present application is intended to include all factors, such as proteinaceous factors, for example, which play a role in the induction or conduction of growth of bone, ligaments, cartilage or other tissues associated with bone or joints. In particular, these growth factors include bFGF, aFGF, EGF (epidermal growth factor), PDGF (platelet-derived growth factor), IGF (insulin-like growth factor), TGF-β. I through III, including the TGF-β. superfamily (BMP-1 through 12, GDF 1 through 12, dpp, 60A, BIP, OF).

Optionally, the growth material may comprise additional osteoconductive bone adhesives, calcium carbonate, fatty acids, lubricants, antiseptic chemicals and/or antibiotics. In this case, other solution excipients such as buffer salts, sugars, anti-oxidants and preservatives to maintain the bioactivity of the growth material and a proper pH of the growth material may be also employed. The additional lubricants and/or the antiseptic and/or the antibiotic will typically be present in the growth material in a predetermined concentration range, which will be dependent upon the particular bone site and application, as well as the specific activity of the antiseptic and/or the antibiotic.

The above description and drawings illustrate preferred embodiments which achieve the objects, features and advantages of the present invention. It is not intended that the present invention be limited to the illustrated embodiments, but rather only by the appended claims.

## Claims

1. A suture anchor, comprising:
a cannulated fixation device provided with side fenestrations; and
an implant for capturing a suture.

2. The suture anchor of claim 1, wherein the implant has a forked distal end for capturing the suture.

3. The suture anchor of claim 2, wherein the implant is configured to allowed rotational insertion without causing excessive twisting and knotting of a suture captured in the forked end.

4. The suture anchor of claim 1, wherein the fixation device has a threaded body.

5. The suture anchor of claim 1, wherein the fixation device has a ribbed body.

6. The suture anchor of claim 1, wherein the fixation device is a cannulated interference screw provided with side fenestrations.
